Europäisches Patentamt

European Patent Office

Office européen des brevets

(19)

(11) Veröffentlichungsnummer: **0 320 528 B1**

(12) # EUROPÄISCHE PATENTSCHRIFT

(45) Veröffentlichungstag der Patentschrift: **08.09.93**

(51) Int. Cl.⁵: **A61K 37/14**, A61K 35/56

(21) Anmeldenummer: **87118765.4**

(22) Anmeldetag: **17.12.87**

Die Akte enthält technische Angaben, die nach dem Eingang der Anmeldung eingereicht wurden und die nicht in dieser Patentschrift enthalten sind.

(54) **Verwendung von Haemocyaninen und Arylphorinen zur Beeinflussung des Immmunsystems und Behandlung von Tumoren.**

(43) Veröffentlichungstag der Anmeldung:
**21.06.89 Patentblatt 89/25**

(45) Bekanntmachung des Hinweises auf die Patenterteilung:
**08.09.93 Patentblatt 93/36**

(84) Benannte Vertragsstaaten:
**AT BE CH DE ES FR GB GR IT LI LU NL SE**

(56) Entgegenhaltungen:

**CHEMICAL ABSTRACTS, Band 96, 1982, Seite 21, Zusammenfassung Nr. 15043x, Columbus, Ohio, US; D.L. LAMM et al.: "Keyhole-limpet hemocyanin and immune ribonucleic acid immunotherapy of murine transitional cell carcinoma", & UROL. RES. 1981, 9(5), 227-30**

(73) Patentinhaber: **Stiefel, Thomas, Dr.**
**Steinkopfstrasse 22**
**D-70184 Stuttgart(DE)**

Patentinhaber: **Porcher, Harald, Dr.**
**Uhlbacher Strasse 61**
**D-70329 Stuttgart(DE)**

Patentinhaber: **Markl, Jürgen, Prof. Dr.**
**Hildastrasse 24**
**D-69115 Heidelberg(DE)**

(72) Erfinder: **Stiefel, Thomas, Dr.**
**Steinkopfstrasse 22**
**D-70184 Stuttgart(DE)**
Erfinder: **Porcher, Harald, Dr.**
**Uhlbacher Strasse 61**
**D-70329 Stuttgart(DE)**
Erfinder: **Markl, Jürgen, Prof. Dr.**
**Hildastrasse 24**
**D-69115 Heidelberg(DE)**

(74) Vertreter: **Patentanwälte Grünecker, Kinkeldey, Stockmair & Partner**
**Maximilianstrasse 58**
**D-80538 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 320 528 B1

CHEMICAL ABSTRACTS, Band 106, 1987, Seite 26, Zusammenfassung Nr. 207290x, Columbus, Ohio, US; P.G. MUNDER et al.: "Studies on the antitumoral effect of keyhole limpet hemocyanine (KLH) in vitro and in vivo", & PRESENT STATUS NON-TOXIC CONCEPTS CANCER, INT. SYMP. 1986 (PUB. 1987), 98-106

CHEMICAL ABSTRACTS, Band 98, 1983, Seite 316, Zusammenfassung Nr. 3449n, Columbus, Ohio, US; D. FORD et al.: "Sensitization of human lymphocytes in vitro. Kinetics and specificity of the response to hemocyanins", & HUM. IMMUNOL. 1982 4(3), 197-208

CHEMICAL ABSTRACTS, Band 96, 1982, Seite 308, Zusammenfassung Nr. 18444q, Columbus, Ohio, US; L. PITZURRA et al.: "Immunogenic effect of hemocyanins of Helix pomatia and Octopus vulgaris hemolymph", & BOLL.-SOC. ITAL. BIOL. SPER. 1981, 57(14), 1517-23

CHEMICAL ABSTRACTS, Band 104, 1986, Seite 540, Zusammenfassung Nr. 67135g, Columbus, Ohio, US; K. RONG et al.: "Optimum hapten-carrier molecular ratios in artificial antigens", & SHENGWU HUAXUE ZAZHI 1985, 1(2), 27-31

CHEMICAL ABSTRACTS, Band 106, 1987, Seite 492, Zusammenfassung Nr. 212066h, Columbus, Ohio, US; M.J.D. VAN TOL et al.: "In vitro detection of antibody secreting cells after in vivo immunization with tetanus toxoid and Helix pomatia hemocyanin", & INT. CONGR. SER. - EXCERPTA MED. 1986, 715 (PROG. IMMUNODEFIC. RES. THER., VOL. 2) 37-45

CHEMICAL ABSTRACTS, Band 105, 1986, Seite 554, Zusammenfassung Nr. 170194v, Columbus, Ohio, US; C. KLEISER et al.: "Immunoprevention of Friend leukemia virus-induced erythroleukemia by vaccination with aggregated gp70", & J. GEN. VIROL. 1986, 67(9), 1901-7

PATENT ABSTRACTS OF JAPAN, Band 6, Nr. 85 (C-103)[963], 22. Mai 1982; & JP-A-57 18 620 (BISEIBUTSU KAGAKU KENKYUKAI) 30-01-1982

**Beschreibung**

Die Erfindung bezieht sich auf die Verwendung von Haemocyaninen und Arylphorinen zur Behandlung von Tumoren.

Haemocyanine sind die Hauptserumproteine zahlreicher Mollusken und Arthropoden. Es sind blaue Kupferproteine und für den Sauerstofftransport verantwortlich. Deren Konzentration im Blut beträgt 20-120 mg/ml. Bei den Mollusken treten sie bei den Schnecken (Klasse Gastropoda), Tintenfischen (Klasse Cephalopoda), Käferschnecken (Klasse Polyplacophora), Elefantenzahnschnecken (Klasse Scaphopoda) und Muscheln (Klasse Bivalvia) auf. Bei den Arthropoden wurden sie nachgewiesen bei den Spinnentieren (Klasse Arachnida), Pfeilschwänzen (Klasse Xiphosura), Krebsen (Klasse Crustacea) und Tausendfüßlern (Klasse Myriapoda).

Arylphorine sind Hauptserumproteine der zu den Arthropoden zählenden Insekten (Klasse Hexapoda) und durch einen hohen Anteil an Phenylalanin und Tyrosin charakterisiert. Sie sind in Konzentrationen bis 60 mg/ml in den Larven und Puppen holometaboler Insekten vorhanden, in niedrigeren Konzentrationen jedoch auch bei adulten Holometabolen sowie bei den Hemimetabolen. Je nach Tierart existieren spezielle Namen für das jeweilige Arylphorin (Drosophorin oder Calliphorin). Arylphorine binden keinen Sauerstoff; verschiedene Funktionen als Stofftransportprotein und als Cuticulakomponente werden diskutiert. Vor kurzem hat sich herausgestellt, beispielsweise durch Untersuchungen mit monoklonalen Antikörpern, daß die Arylphorine eindeutig strukturhomolog zu den Arthropodenhaemocyaninen sind und wahrscheinlich deren Abkömmlinge darstellen.

Haemocyanine und Arylphorine werden in speziellen Zellen synthetisiert. Diese Zellen flottieren zumindest während ihrer Biogenese nicht frei in der Blutbahn, sondern sind mit bestimmten Geweben assoziiert, die mit dem Blut in Kontakt stehen (Herzmuskelsepten, Mitteldarmdrüse, Fettkörper oder Augensinus). Die Haemocyanine und Arylphorine werden zunächst in diesen Zellen angereichert und schließlich ins freie Blut abgegeben. Das native Schneckenhaemocyanin ist zylinderförmig und erreicht mit 35 nm Durchmesser die Größe eines Poliovirus. Die übrigen Molluskenhaemocyanine sind halb so groß. Die Arthropodenhaemocyanine und die Arylphorine basieren strukturell auf einem Würfel von 10 nm Kantenlänge. Bei den Arylphorinen kann dieser Würfel halbiert sein, während er bei den Haemocyaninen sehr oft oligomerisiert. Das größte Arthropodenhaemocyanin (Pfeilschwänze) besteht aus 8 Würfeln und hat eine Kantenlänge von 25 nm, was etwa der Größe eines Ribosoms entspricht.

Arthropodenhaemocyanine und Arylphorine bestehen aus bis zu 8 immunologisch verschiedenen Untereinheiten mit Molekulargewichten um 70-80.000. Der 10 nm-Grundwürfel ist ein Hexamer aus solchen Untereinheiten. In den oligohexameren Haemocyaninen spielt jeder Untereinheiten-Typ eine spezifische Strukturrolle. Es existieren mehrere komplette Sequenzanalysen sowie ein detailliertes Röntgenstrukturmodell. Bei den Arylphorinen sind zahlreiche Details der Genstruktur bekannt.

Haemocyanine und Arylphorine lassen sich durch Dialyse gegen alkalischen pH und Entzug divalenter Kationen in Untereinheiten zerlegen. Bei den Mollusken besteht diese Untereinheit aus 7-8 globulären Domänen, von denen jede ein Molekulargewicht um 55.000 besitzt. Die Domänen sind immunologisch sehr verschieden. Eine davon wurde bisher komplett sequenziert. Jede trägt ein aktives, sauerstoffbindendes Zentrum, bestehend aus 2 Kupferatomen. Im nativen Molekül sind bis zu 160 solcher Domänen vorhanden. Ihre Anordnung in der Quartärstruktur ist detailliert bekannt.

Aufgrund der geschilderten strukturellen Heterogenität und seiner vollkommen xenogenen Natur, da Mollusken und Arthropoden als Protostomier von den Wirbeltieren seit mindestens 600 Millionen Jahren getrennt sind, sind Haemocyanine die stärksten bekannten Antigene. Sie führen beim Säuger zur Bildung eines sehr starken Antiserums, verschieben das T4/T8-Verhältnis zugunsten der T4-Helferzellen und führen am Applikationsort zu einem lokalen Erythem und zu einer Makrophageninvasion. Dies wird beim "Keyhole Limpet haemocyanin" (KLH) seit vielen Jahren in der immunologischen Forschung ausgenützt.

Es ist bekannt, daß KLH zytotoxische T-Zellen induziert und deutliche Antitumoreffekte in vitro und in verschiedenen Tiermodellen in vivo zeigt. Dies wurde am KLH schon öfter demonstriert. Eine klinische Behandlung mit KLH führt zu einem signifikanten Absinken der Rezidivrate beim oberflächlichen Harnblasenkarzinom, wobei unangenehme Nebenwirkungen niemals beobachtet werden, und auch andere Tumortypen positiv beeinflußt werden (Jurincic, C.D. et al.; Uroscop, Information und Fortbildung in der Urologie, Heft 1, 1986). Zwar ist der exakte Ablauf des Wirkmechanismus von KLH auf die Blasenkarzinomzellen noch nicht ganz klar; es zeigt sich jedoch, daß der Angriffspunkt sich vollständig von den bisher existierenden Chemotherapie-Ansätzen unterscheidet. Die Ergebnisse der Chemotherapien sind teilweise widersprüchlich. Während zytotoxische Medikamente sämtliche urothelialen Zellen angreifen, stimuliert KLH die Makrophagen des gesamten Organismus (Curtis, J.E. et al. "Antigen Dose in Human Immune Response Relationships in the Human Immune Response to Keyhole-Limpet Haemocyanine". Journal of laboratory in

3

Clinical Medicine 78,61 (1971)). Curtis konnte zeigen, daß KLH sowohl eine primäre zellvermittelte, verzögerte Hypersensibilität vermittelt als auch eine primäre Antikörper-Antwort im Menschen.

Dieses potente Antigen wirkt daher als ein unspezifisches Stimulanz, das T-Lymphozyten und Lymphokin-produzierende Makrophagen aktiviert.

Es war Aufgabe der Erfindung, eine weitere Möglichkeit zur Behandlung von Tumoren bereitzustellen.

Die Aufgabe wird durch die Verwendung von Haemocyanin, ausgenommen Keyhole-Limpet Haemocyanin, und/oder Arylphorin zur Herstellung eines Medikaments zur Behandlung von Tumoren gelöst.

Bei der Überprüfung verschiedenster Haemocyanine und Arylphorine in ihrer Wirkung auf die Wachstumshemmung von Tumoren hat sich überraschenderweise gezeigt, daß sich die Haemocyanine und Arylphorine verschiedener Tiergruppen immunologisch deutlich unterscheiden und verschieden stark ausgeprägte antitumorale Wirkung zeigen. Durch diesen Umstand können neben dem Keyhole-Limpet Haemocyanin weitere wertvolle Substanzen der Stoffklasse der Haemocyanine und Arylphorine für die Bekämpfung von Tumoren zur Verfügung gestellt werden. Insbesondere können auch andere Tumortypen, als das für die Anwendung des Keyhole-Limpet Haemocyanins bekannte Harnblasenkarzinom, mit den genannten Substanzen behandelt werden. Wie bereits erwähnt, sind die antitumoralen Wirkmechanismen der Haemocyanine nicht bekannt. Arthropoden-Haemocyanine verfügen jedoch über eine Teilstruktur, die eine auffällige Ähnlichkeit mit dem Grundaufbau der Immunglobuline und der MHC-Proteine zeigt. Dies könnte in Verbindung mit dem bereits diskutierten Einfluß auf das Immunsystem der Grund für die Wirkungsweise sein. Die antitumorale Wirksamkeit verschiedener Haemocyanine wurde am Methylcholantreninduzierten Fibrosarkom der Maus geprüft. Die gefundenen Daten lassen den Schluß zu, daß Haemocyanine und Arylphorine die Immunkompetenz prophylaktisch steigern.

Das Haemocyan in oder Arylphorin wird aus Geweben von Mollusken oder Arthropoden isoliert, wobei die jeweiligen einzelnen Arten der Mollusken oder Arthropoden ausgewählt werden aus einer Gruppe, die die Gattungen Eurypelma, Limulus, Astacus, Carcinus, Calliphora, Helix und Octopus umfaßt. Die genannten Arten sind sicherlich nicht die einzigen Arten unter den Mollusken oder Arthropoden, aus denen Haemocyanine oder Arylphorine isoliert werden können, die eine das Tumorwachstum hemmende Wirkung aufweisen.

Die Ergebnisse der aus den genannten Gattungen überprüften Substanzen sind in Tabelle 1 unter anderem aufgeführt. Eine antitumorale Wirkung ist in jedem Fall vorhanden, wobei diese jedoch bei den nativen Komponenten deutlich stärker ist als beispielsweise bei solchen Komponenten, die mit SDS denaturiert wurden.

Für die Isolierung der Haemocyanine oder Arylphorine der genannten Gattungen wird das Blut der jeweiligen Mollusken oder Arthropoden durch Punktion von Extremitäten, Blutlakunen oder des Herzens gewonnen. Die Blutzellen werden abzentrifugiert, wodurch die Gerinnung verhindert wird. Aus dem so gewonnenen überstand, dem Plasma, werden die Haemocyanine bzw. Arylphorine durch Pelletieren in der präparativen Ultrazentrifuge von kleineren Haemolymphekomponenten gereinigt und anschließend in PBS wieder gelöst. Die resultierende Haemocyanin- oder Arylphorinpräparation ist bis zu 95 % gereinigt. Eine 100%-ige Reinheit erreicht man durch anschließende Chromatographie über einen Anionenaustauscher. Weitere Reinigungen des Arylphorins können durch Lösen von Bindungen des Arylphorins an andere Serumproteine, beispielsweise bei einem pH-Wert von 9,6 (Glycin/OH-Puffer, 10 mM EDTA) erreicht werden, wobei das Arylphorin dann in Untereinheiten dissoziiert wird. Durch anschließende Anionenaustauschchromatographie erhält man 100 % reines Arylphorin, das durch Dialyse gegen neutralen Puffer (Tris/Glycin) wieder zum Hexamer rekonstituiert werden kann.

Vorzugsweise stammen die erfindungsgemäß verwendeten Haemocyanine aus den Arten Eurypelma californicum, Helix pomatia, Octopus vulgaris, Astacus fluviatilis, Carcinus maenas oder Limulus polyphemus.

Ein bevorzugtes Arylphorin stammt aus Calliphora erythrocephala.

Präparationen aus den genannten Tierarten werden in 0,1 M Tris/HCl-Puffer vom pH 7,5 zur Verfügung gestellt. Als Dosis wurden 3 x 100 $\mu$g in 0,5 ml PBS pro Tier und Tag subkutan auf zwei Stellen verteilt (Leiste bzw. axillär) appliziert. Als Tumorzellen wurden syngene Met-A-Aszites-Tumorzellen verwendet aus einer in vivo-Passage. Die Zellen waren tryptophanblau geprüft (2 % positiv). Es wurden 1 x $10^5$ Zellen i.c. implantiert. Die Behandlung mit einem Haemocyanin aus den oben vorzugsweise genannten Tierarten erfolgte an den Tagen +2, +5 und +7 mit jeweils 100 $\mu$g subkutan. In Tabelle 1 sind die Ergebnisse bzgl. des Tumorvolumens und der überlebensrate nach 4 Wochen dargestellt. Wie die Tab.1 zeigt, hat in der unbehandelten Kontrollgruppe kein Tier von 10 Tieren den Zeitraum von 28 Tagen nach Tumorimplantation überlebt, während in den Haemocyanin-behandelten Gruppen zwischen 6 und 2 von 10 Tieren diesen Zeitraum überlebten und die Tumorvolumina nach 28 Tagen zwischen 11 und 34 % der unbehandelten Kontrollgruppe betrugen.

Der Wiederanstieg der Tumorvolumina nach 28 Tagen ist darauf zurückzuführen, daß die Behandlung bereits nach 7 Tagen abgebrochen wurde, weil ein immunologischer Behandlungserfolg erfahrungsgemäß innerhalb von 2 Wochen sichtbar wird.

Auch Untereinheiten, die antigene Domänen der vollständigen Haemocyanine oder Arylphorine enthalten, können vorzugsweise verwendet werden. Wie bereits einleitend erwähnt, sind die Untereinheiten der Mollusken-Haemocyanine aus 7 bis 8 immunologisch verschiedenen Domänen zusammengesetzt, von denen jede ein Molekulargewicht um 55.000 besitzt. Die Arthropodenhaemocyanine und Arylphorine können bis zu 8 immunologisch verschiedene Untereinheiten mit Molekulargewichten um 70-80.000 enthalten. Da die Untereinheiten immunologisch sehr verschieden sind, war es überraschend, daß alle untersuchten Untereinheiten Wirksamkeit zeigten.

Bevorzugt sind die Untereinheiten a, d und/oder e des Haemocyanins aus Eurypelma californicum. Wie Tabelle 1 zeigt, ist insbesondere die Wirkung der Untereinheit d nicht in allen ihren Regionen gleich.

Die vorzugsweise Verwendung der Domänen d und/oder g des βc-Haemocyanins aus Helix pomatia zeigt ebenfalls einen Hemmechanismus auf das Wachstum von Tumoren nach 14 und 21 Tagen.

Die Untereinheit a des Haemocyanins aus Eurypelma californicum kann weiterhin vorzugsweise chymotryptisch gespalten werden. Aus dem Spaltgemisch kann sodann ein Peptidfragment, bestehend aus 23 Aminosäuren, erhalten werden, das prädestiniert für eine Wirkung der beanspruchten Art ist.

Das genannte, 23 Aminosäuren enthaltende Peptid kann vorzugsweise an Haemocyanin aus Carcinus oder Octopus oder an Arylphorin aus Calliphora gebunden werden. Die Wirkung dieser gekoppelten, 23 Aminosäuren enthaltenden Peptiduntereinheit ist in Tab.1 dargestellt, und es zeigt sich, daß insbesondere diese Ausführungsform der Erfindung einen enormen Effekt zeigt, da neben einer drastischen Reduzierung der Tumorgröße eine hohe Überlebensrate von jeweils 7 bzw. 8 aus 10 Tieren zu beobachten ist.

Eine Verstärkung der beanspruchten Wirkungen wird weiterhin erreicht, wenn komplette Haemocyanine oder Arylphorine oder die geschilderten Untereinheiten entweder in Mischung oder chemisch oder physikalisch mit weiteren Bestandteilen gekoppelt werden, die aus Viren oder Virenbestandteilen, Bakterien oder Bakterienbestandteilen, Pilzen oder deren Bestandteilen sowie tierischen Parasiten oder deren Bestandteilen bestehen können.

Eine besonders intensive Wirkung wird dabei erreicht, wenn die genannten Substanzen an Meth-A-Aszites-Tumorzellfragmente gekoppelt werden. Wie die Tab.1 zeigt, wird mit dieser Kopplung ebenfalls ein enormer tumorreduzierender und die überlebenschance steigernder Effekt erzielt.

Die Erfindung wird im weiteren anhand der Beispiele näher erläutert:

Beispiel 1

Isolierung von Haemocyaninen und Arylphorinen

Mollusken- bzw. Arthropodenblut wird durch Punktion von Extremitäten, Blutlakunen oder des Herzens gewonnen. Die Blutzellen werden abzentrifugiert, was die Gerinnung verhindert. Aus dem überstand (=Plasma) werden Haemocyanine bzw. Arylphorine durch Pelletieren in der präparativen Ultrazentrifuge von kleineren Haemolymphekomponenten gereinigt und anschließend in PBS (phosphate buffered saline) wieder gelöst. Die resultierende Haemocyaninpräparation ist zu mehr als 95 % sauber. 100 % Reinheit wird durch anschließende Chromatographie über einen Anionenaustauscher erreicht. Pelletiertes Arylphorin ist stärker kontaminiert, da es andere Serumproteine locker bindet. Diese Bindungen werden gebrochen, indem das Arylphorin bei pH 9.6 (Glycin/OH-Puffer, 10 mM EDTA) in Untereinheiten dissoziiert wird. Eine anschließende Anionenaustauschchchromatographie liefert 100 % reines Arylphorin, das durch Dialyse gegen neutralen Puffer (Tris/Glycin) wieder zum Hexamer rekonstituiert werden kann.

Beispiel 2:

Die antitumorale Wirksamkeit verschiedener Haemocyanine wurde am Methylcholanthren-induzierten Fibrosarkom der Maus geprüft. Das Modell ist z.B. beschrieben in: Munder, P.G. et al: "Antitumorale Wirkung xenogener Substanzen in vivo und in vitro", Onkologie 5, 4-7 (1982). Verwendet wurden das Arylphorin der Schmeißfliege Calliphora erythrocephala, sowie die Haemocyanine folgender Tiere: Helix pomatia (Weinbergschnecke), Octopus vulgaris (Krake), Astacus fluviatilis (Flußkrebs), Carcinus maenas (Strandkrabbe), Limulus polyphemus (Pfeilschwanz) und Eurypelma californicum (Vogelspinne). Sämtliche Präparationen lagen in 0.1 M Tris/HCl-Puffer vom pH 7.5 vor. Als Dosis wurden 3x100 μg in 0,5 ml PBS pro Tier und Tag s.c. auf zwei Stellen verteilt (Leiste bzw. axillär) appliziert. Als Tumorzellen wurden syngene Meth-A-Aszites-Tumorzellen verwendet aus einer in vivo-Passage. Die Zellen waren tryptophanblau geprüft (2 % positiv). Es wurden 1x10$^5$ Zellen i.c. implantiert. Die Behandlung mit Haemocyanin erfolgte an Tag +2, +5 und +7 mit jeweils 100 µg s.c.. In Tabelle 1 sind die Ergebnisse bezüglich des Tumorvolumens und der überlebensrate nach 4 Wochen dargestellt. Wie man erkennt, hat in der unbehandelten Kontrollgruppe kein Tier von 10 Tieren den Zeitraum von 28 Tagen nach Tumorimplantation überlebt, während in den Haemocyanin-Gruppen zwischen 6 und 2 von 10 Tieren diesen Zeitraum überlebten und die Tumorvolumina nach 28 Tagen zwischen 11 und 34 % der unbehandelten Kontrollgruppe betrugen. Der Effekt war bei den Crustaceen-Haemocyaninen am geringsten, was auf die geringere Zahl an Untereinheiten-Typen und damit an Antigen-Determinanten zurückgeführt werden kann (Markl, J.: "Evolution and function of structurally diverse subunits in the respiratory protein haemocyanin from arthropods", Biol. Bull. 171, 90-115 (1986)).

Beispiel 3:

a) Die Untereinheiten a, d, und e des Haemocyanins der Vogelspinne Euroypelma californicum sowie die Domänen d und g des βc-Haemocyanins der Weinbergschnecke Helix pomatia wurden nach publizierten Verfahren im nativen Zustand isoliert (Markl, J. et al.: "Hemocyanins in spiders VI. Comparison of the polypeptide chain of Eurypelma californicum hemocyanin", Hoppe-Seyler's Z. Physiol. Chem. 360, 639-650 (1979); Lontie, R.: "Components, functional units, and acitve sites of Helix pomatia haemocyanin", Life Chem.Rep.Suppl. 1, 109-120 (1983)) und ihre antitumorale Effektivität im Meth-A-Sarkom-Modell getestet.

b) Die Untereinheit d der Vogelspinne sowie die Domäne d der Schnecke wurden auch im SDS-denaturierten Zustand eingesetzt (Denaturierung in 2 % SDS; das SDS wurde vor der Applikation in die Mäuse durch Dialyse auf 0,1 % reduziert).

c) Die native Untereinheit d der Vogelspinne wurde durch limitierte Trypsinolyse in zwei nahezu gleich große Fragmente vom Molekulargewicht 34.000 und 37.000 gespalten und die Fragmente isoliert wie beschrieben (Schartau et al.: "Hemocyanins in spiders, XIX. Complete amino-acid sequence of subunit d from Eurypelma californicum haemocyanin, and comparison to chain e. Hoppe-Seyler's Z. Physiol. Chem. 364, 1383-1409 (1983)). Diese beiden denaturierten, als dTn34 und dTn37 bezeichneten Peptidfragmente wurden ebenfalls im Meth-A-Sarkom-Modell getestet.

Alle Ergebnisse sind in Tabelle 1 mit aufgeführt. Eine antitumorale Wirkung ist in jedem Fall vorhanden, wobei diese jedoch bei den nativen Komponenten deutlich stärker ist als bei den denaturierten. Bei Untereinheit d der Vogelspinne war zwischen intakter Polypeptidkette und dem C-terminalen Peptidfragment dTn37 kein signifikanter Unterschied zu erkennen, während das N-terminale Peptidfragment dTn34 deutlich abfiel. Insgesamt ist die Wirkung isolierter Komponenten jedoch zweifellos schwächer als diejenige kompletten Haemocyanins aus Beispiel 2.

d) Schließlich wurde mit Hilfe des kürzlich vorgestellten, sequenzgebundenen monoklonalen Antikörpers Ec-8 (Markl, J.: "Characterization of monoclonal antibodies to tarantula haemocyanin", Verh. Dtsch. Zool. Ges. 80, in press (1987)), der spezifisch gegen die Untereinheit a aus der Vogelspinne gerichtet ist, aus dem chymotryptischen Spaltgemisch von Untereinheit a durch Immun-Affinitätschromatographie ein Peptid aus 23 Aminosäuren isoliert, das die dem Ec-8 entsprechende Antigendeterminante trägt. Dieses isolierte Antigen wurde an die Haemocyanine aus Carcinus und Octopus sowie an das Arylphorin von Calliphora durch Standardverfahren (Palreyman, J.W. et al.: "Guidelines of the production of polypeptide specific antisera using small synthetic oligopeptides as immunogens", J.Immunol.Meth.75, 383-393 (1984)) gekoppelt und in dieser Form im Meth-A-Sarkom-Modell getestet. Die antitumorale Effektivität der drei Trägermoleküle, auf denen das Ec-8-Epitop nicht vorkommt, wurde dadurch erhöht (Tab.1).

Beispiel 4:

Möglichkeiten, die Applikation von Haemocyaninen bzw. Arylphorinen diagnostisch auszuwerten, wurden mit den Haemocyaninen aus der Vogelspinne Eurypelma, der Strandkrabbe Carcinus und des Octopus sowie mit dem Arylphorin der Schmeißfliege Calliphora getestet. Bei dem Test handelte es sich um einen B-Zell-Funktionsassay, der über die Messung der Antikörpertiter nach subcutaner Injektion als Neoantigen in Meerschweinchen erfolgte. Dabei wurden jeweils zwei Kontrolltiere mit zwei Tieren verglichen, deren Immunreaktionen durch Cyclosporin (6.25 mg/kg Körpergewicht, 2 x täglich, über 30 Tage) unterdrückt waren. Zum Verfahren siehe Amlot P.L. et al. ("Human immune response in vivo to protein (KLH) and polysaccharide (DNP-Ficoll) neoantigens: normal subjects compared with bone marrow transplant patients on cyclosporine", Clin.Exp.Immunol.64, 125-135 (1986)). Der Antikörpertiter wurde über einen ELISA mit 1 ng Haemocyanin oder Arylphorin pro Microtiter-Well bestimmt, wobei anti-(Meerschweinchen IgM) und anti-(Meerschweinchen-IgG) - jeweils gekoppelt mit Alkalischer Phosphatase - als zweites Antiserum verwendet wurde. Die vier getesteten Substanzen erfüllen alle von Amlot P.L. et al. geforderten Kriterien und erwiesen sich als geeignet, die Immundefizienz zu diagnostizieren. Die sehr gute Verwendbarkeit des KLH zur Diagnose von Immundefizienzen und anderen immunologischen Parametern gilt somit überraschenderweise offensichtlich für alle Haemocyanine und Arylphorine.

Beispiel 5:

Die Haemocyanine aus der Vogelspinne Eurypelma, der Strandkrabbe Carcinus und des Kraken Octopus sowie das Arylphorin der Schmeißfliege Calliphora wurden durch in der Literatur beschriebene Verfahren (Palfreyman et al.: "Guidelines of the production of polypeptide specific antisera using small synthetic oligopeptides as immunogens", J. Immunol. Meth. 75, 383-393 (1984) sowie Jan Dahmen et al. "Synthesis of spacer-arm, lipid, and ethyl glycosides of the terminal trisaccharide ($\alpha$-D-Gal(1→4)-$\beta$-D-Gal-(1→4)-$\beta$-D-GlcNAc) portion of the blood-group P$_1$ antigen: preparation of neoglycoproteins", Carbohydrate Research 129, 63-71 (1984); Jan Dahmen et al.: "Synthesis from pullulan of spacer-arm lipid, and ethyl glycosides of a tetrasaccharide ($\alpha$→D-Glc-(1→6)-$\alpha$-D-Glc-(1→4)-$\alpha$-D-Glc(1→4)-D-Glc) found in human urine; preparation of neoglycoproteins", Carbohydrate Research 127, 27-33 (1984); Jan Dahmen et al.: "Synthesis of spacer-arm, lipid, and ethyl glycosides of the trisaccharide portion ($\alpha$-D-Gal-(1→4)-$\beta$-D-Gal-(1→4)-$\beta$-D-Glc) of the blood-group P$^k$ antigen: preparation of neoglycoproteins", Carbohydrate Research 127, 15-25 (1984); Jan Dahmen et al.: "2-bromoethyl glycosides in glycoside synthesis: Preparation of glycoproteins containing $\alpha$-L-Fuc-(1→2)-D-Gal and $\beta$-D-Gal-(1→4)-D-GlcNAc", Carbohydrate Research 125, 237-245 (1984); R.U. Lemieux, D.A. Baker and D.R. Bundle: "A methodology for the production of carbohydrate-specific antibody", Can.J.Biochem. 55, (1977); J.D. Aplin, J.C. Wriston, Jr.: "Preparation, properties, and applications of carbohydrate conjugates of proteins and lipids", CRC Critical Reviews in Biochemistry, pp 259 (1981); B.M. Pinto and D.R. Bundle: "Preparation of glycoconjugates for the use as artificial antigens: A simplified procedure",Carbohydrate Research 124, 313-318 (1983)) mit folgenden teils kommerziell erhältlichen, teils aus anderen Quellen stammenden Substanzen gekoppelt:
a) Gangliosid GM2/GD3
b) Chymotryptischer Fragmentcocktail von Meth-A-Aszites-Tumorzellen
c) Virusfragment (Herpes-Viren)
d) Bakterienfragment (BCG, Muramyldipeptide)
e) Pilzfragmente von Cryptococcus neoformans
f) Schistosomula Oberflächenantigen von Schistosoma mansoni.
Durch Applikation in Mäuse (dreimalige Standardimmunisierung über 6 Wochen) wurde jeweils ein starkes, haptenspezifisches Antiserum erhalten, auch bei den syngenen Meth-A-Aszites-Fragmenten. Um einen therapeutischen Kombinationseffekt nachzuweisen, der einerseits über die immunmodulatorischen und antitumoralen Mechanismen des Haemocyanins selbst und andererseits über spezifische anti-Hapten-Antikörper wirken sollte, wurde folgendermaßen vorgegangen: Euroypelma-Haemocyanin wurde mit Tumorzellfragmenten aus Meth-A-Aszites gekoppelt und im Meth-A-Sarkom-Tumormodell getestet. Das Ergebnis in Tabelle 1 zeigt eine Steigerung des Therapieeffektes gegenüber der Behandlung mit reinem Eurypelma-Haemocyanin. Nach 28 Tagen hatten acht von zehn Mäusen überlebt. Damit hat sich die Kombination von Haemocyanin als unspezifisches Adjuvans mit einer spezifischen antigenen Komponente als wirkungsvoller Impfstoff erwiesen (zum Prinzip vergleiche Schirrmacher, V.: "Postoperative activation of tumor-specific T-cells as a means to achieve immune control of minimal residual disease", General Motors Cancer Research Foundation. Accomplishments in cancer research 1986, Fortner and Roads (eds.), pp 218-232).

Beispiel 6:

Zum Nachweis einer allgemeinen Stärkung des Immunsystems wurden 10 Mäuse im Zeitraum von 6 Wochen durch dreimalige subkutane Applikation von jeweils 0,1 mg Helix pomatia-Haemocyanin immunisiert. Nach weiteren 3 Wochen wurden wie im Beispiel 2 Meth-A-Aszites-Tumorzellen implantiert, und die Mäuse anschließend mit Carcinus-Haemocyanin therapiert (zwischen Mollusken- und Arthropodenhaemocyanin existieren keine immunologischen Kreuzreaktionen). Umgekehrt wurden 10 Mäuse mit Calliphora-Arylphorin vorbehandelt und nach Tumorzellimplantation mit Octopus-Haemocyanin therapiert. In beiden Fällen war der Therapieerfolg deutlich besser als bei den Tieren ohne Vorbehandlung, wie sich aus Tabelle 1 ergibt. Messungen im Rahmen von Immunisierungsversuchen von Meerschweinchen mit den Haemocyaninen aus Limulus, Eurypelma, Carcinus, Octopus und Helix sowie mit Calliphora-Arylphorin zeigten eine massive Expansion der Makrophagen und der T4-Helferzellen nach Applikation. Zusammengenommen

7

lassen die Daten den Schluß zu, daß Haemocyanin und Arylphorin die Immunkompetenz prophylaktisch steigert.

Beispiel 7:

Die Verwendung der Haemocyanine aus Carcinus und Octopus sowie des Arylphorins aus Calliphora als Haptenträger (Ec-8-spezifisches Peptidfragment als Hapten) mit dem Ziel, eine Reaktion des zellulären Immunsystems zu erreichen, wurde bereits im letzten Abschnitt des Beispiels 3 geschildert. Parallel dazu wurde dieses Hapten mit den drei Trägern in jeweils 2 Meerschweinchen gespritzt (3 x im Zeitraum von 6 Wochen jeweils 0,1 mg subcutan). Das Ergebnis war in jedem Fall ein starkes Antiserum nicht nur gegen das Trägermolekül, sondern auch gegen das Hapten. Zwei nur mit dem Hapten gespritzte Kontrolltiere zeigten dagegen eine negative Reaktion. Die Reaktion des Haptens konnte bei den positiven Tieren immunologisch leicht von der des Trägermoleküls differenziert werden, da es sich um ein vogelspinnen-spezifisches Epitop handelt. Die gegen das Hapten gerichtete IgG-Fraktion wurde durch Affinitätsreinigung an Vogelspinnen-Haemocyanin-gekoppelter BrCN-Sepharose CL-4B isoliert und reagierte im Immunoblot hochspezifisch mit der Untereinheit a. Weitere Daten zu diesem Thema liefern die Beispiele 4 und 5.

EP 0 320 528 B1

| Haemocyanin/ Arylphorin-Quelle | 7 Tage | | 14 Tage | | 21 Tage | | 28 Tage | | überlebende Tiere |
|---|---|---|---|---|---|---|---|---|---|
| | Tumorvolumen ccm | % | Tumorvolumen ccm | % | Tumorvolumen ccm | % | Tumorvolumen ccm | % | |
| Eurypelma | 13.7 | 107 | 6.4 | 27 | 5.6 | 11 | 20.1 | 16 | 5/10 |
| Limulus | 13.4 | 105 | 8.1 | 34 | 5.1 | 10 | 13.8 | 11 | 6/10 |
| Astacus | 13.7 | 106 | 9.3 | 39 | 9.2 | 18 | 30.1 | 24 | 2/10 |
| Carcinus | 17.8 | 139 | 13.1 | 55 | 16.4 | 32 | 42.6 | 34 | 5/10 |
| Calliphora | 13.9 | 109 | 10.0 | 42 | 12.8 | 25 | 38.8 | 31 | 3/10 |
| Helix | 12.2 | 95 | 7.6 | 32 | 7.1 | 14 | 21.3 | 17 | 6/10 |
| Octopus | 14.6 | 114 | 6.9 | 29 | 5.6 | 11 | 17.5 | 14 | 5/10 |
| Eurypelma a | 13.8 | 108 | 17.6 | 74 | 27.6 | 54 | 80.2 | 64 | 2/10 |
| Eurypelma d | 13.1 | 102 | 19.8 | 83 | 34.8 | 68 | 101.5 | 81 | 0/10 |
| Eurypelma e | 10.6 | 83 | 15.7 | 66 | 24.5 | 48 | 68.9 | 55 | 2/10 |
| Helix d | 12.3 | 96 | 15.5 | 65 | 28.6 | 56 | 89.0 | 71 | 0/10 |
| Helix a | 14.7 | 115 | 17.1 | 72 | 31.2 | 61 | 77.7 | 62 | 1/10 |
| Eurypelma d SDS | 15.6 | 122 | 20.5 | 86 | 36.3 | 71 | 104.0 | 83 | 0/10 |
| Helix d SDS | 15.1 | 118 | 18.8 | 79 | 34.8 | 68 | 99.0 | 79 | 0/10 |
| Eurypelma d Tn 34 | 13.2 | 103 | 23.3 | 98 | 53.7 | 105 | 150.4 | 120 | 0/10 |
| Eurypelma d Tn 37 | 12.7 | 99 | 17.6 | 74 | 36.8 | 72 | 111.5 | 89 | 0/10 |
| Carcinus x Ec-8-Antigen | 13.7 | 107 | 8.1 | 34 | 7.2 | 14 | 21.3 | 17 | 6/10 |
| Calliphora x Ec-8-Antigen | 17.2 | 134 | 10.0 | 42 | 11.2 | 22 | 35.1 | 28 | 3/10 |
| Octopus x Ec-8-Antigen | 11.8 | 92 | 5.5 | 23 | 4.1 | 8 | 15.0 | 12 | 6/10 |
| Eurypelma x Meth-A-Fragmente | 14.1 | 110 | 4.5 | 19 | 3.6 | 7 | 11.3 | 9 | 8/10 |
| Carcinus (präimmunisiert mit Helix) | 11.1 | 87 | 7.6 | 32 | 5.6 | 11 | 15.0 | 12 | 7/10 |
| Octopus (präimmunisiert mit Calliphora) | 13.8 | 108 | 6.4 | 27 | 4.6 | 9 | 16.3 | 13 | 8/10 |
| Kontrolle | 12.8 | 100 | 23.8 | 100 | 51.1 | 100 | 125.3 | 100 | 0/10 |

TABELLE 1

**Patentansprüche**

**Patentansprüche für folgende Vertragsstaaten : AT, BE, CH, DE, FR, GB, GR, IT, LI, LU, NL, SE**

1. Verwendung von Haemocyanin, ausgenommen Keyhole-Limpet Haemocyanin, und/oder Arylphorin zur Herstellung eines Medikaments zur Behandlung von Tumoren.

2. Verwendung nach Anspruch 1, **dadurch gekennzeichnet,** daß das Haemocyanin oder Arylphorin aus Geweben von Mollusken oder Arthropoden isoliert wird, wobei die Mollusken oder Arthropoden ausgewählt werden aus den Gattungen Eurypelma, Limulus, Astacus, Carcinus, Calliphora, Helix und Octopus.

3. Verwendung nach Anspruch 2, **dadurch gekennzeichnet,** daß ein Haemocyanin aus Eurypelma californicum (Vogelspinne) verwendet wird.

4. Verwendung nach Anspruch 2, **dadurch gekennzeichnet,** daß ein Haemocyanin aus Helix pomatia (Weinbergschnecke) verwendet wird.

5. Verwendung nach Anspruch 2, **dadurch gekennzeichnet,** daß ein Haemocyanin aus Octopus vulgaris (Krake) verwendet wird.

6. Verwendung nach Anspruch 2, **dadurch gekennzeichnet,** daß ein Haemocyanin aus Astacus fluviatilis (Flußkrebs) verwendet wird.

7. Verwendung nach Anspruch 2, **dadurch gekennzeichnet,** daß ein Haemocyanin aus Carcinus meanas (Strandkrabbe) verwendet wird.

8. Verwendung nach Anspruch 2, **dadurch gekennzeichnet,** daß ein Haemocyanin aus Limulus polyphemus (Pfeilschwanz) verwendet wird.

9. Verwendung nach Anspruch 2, **dadurch gekennzeichnet,** daß ein Arylphorin aus Calliphora erythrocephala verwendet wird.

10. Verwendung nach mindestens einem der vorhergehenden Ansprüche, **dadurch gekennzeichnet,** daß Untereinheiten eines Haemocyanins oder Arylphorins, vorzugsweise solche, die antigene Domänen enthalten, verwendet werden.

11. Verwendung nach Anspruch 10, **dadurch gekennzeichnet,** daß Untereinheiten eine der globulären Domänen der Haemocyanine der Mollusken mit einem Molekulargewicht von etwa 55.000 sind.

12. Verwendung nach Anspruch 10, **dadurch gekennzeichnet,** daß die Untereinheiten des Haemocyanins oder Arylphorins der Arthropoden Molekulargewichte von etwa 70.000 bis 80.000 aufweisen.

13. Verwendung nach Anspruch 12, **dadurch gekennzeichnet,** daß die Untereinheiten $\underline{a}$, $\underline{d}$ und/oder $\underline{e}$ des Haemocyanins aus Eurypelma californicum verwendet werden.

14. Verwendung nach Anspruch 13, **dadurch gekennzeichnet,** daß ein Peptidfragment, bestehend aus 23 Aminosäuren, verwendet wird, das durch chymotryptische Spaltung der Untereinheit $\underline{a}$ erhalten wird.

15. Verwendung nach Anspruch 14, **dadurch gekennzeichnet,** daß das Peptidfragment, bestehend aus 23 Aminosäuren, an Haemocyanin aus Carcinus oder Octopus oder an Arylphorin aus Calliphora gebunden ist.

16. Verwendung nach Anspruch 12, **dadurch gekennzeichnet,** daß die Domänen $\underline{d}$ und/oder $\underline{a}$ des $\beta$c-Haemocyanins aus Helix pomatia verwendet werden.

17. Verwendung nach mindestens einem der vorhergehenden Ansprüche, **dadurch gekennzeichnet,** daß das Haemocyanin und/oder Arylphorin mit Viren oder Virenbestandteilen, Bakterien oder Bakterienbestandteilen, Pilzen oder Pilzbestandteilen oder tierischen Parasiten oder deren Bestandteilen Strukturähnlichkeiten aufweisen oder mit diesen gemischt oder chemisch oder physikalisch gekoppelt werden.

18. Verwendung nach mindestens einem der vorhergehenden Ansprüche, **dadurch gekennzeichnet,** daß vor Applikation eine physikalische oder chemische Kopplung des Haemocyanins oder Arylphorins mit Antigenen zur Verstärkung der Antitumorwirkung erfolgt.

19. Verwendung nach Anspruch 18, **dadurch gekennzeichnet,** daß die Kopplung an Meth-A-Aszites-Fragmenten erfolgt.

**Patentansprüche für folgenden Vertragsstaat : ES**

1. Verfahren zur Herstellung eines Medikaments, enthaltend Haemocyanin, ausgenommen Keyhole-Limpet Haemocyanin, und/oder Arylphorin zur Behandlung von Tumoren, wobei das Verfahren in an sich bekannter Weise durchgeführt wird.

2. Verfahren nach Anspruch 1, **dadurch gekennzeichnet,** das daß Haemocyanin oder Arylphorin aus Geweben von Mollusken oder Arthropoden isoliert wird, wobei die Mollusken oder Arthropoden ausgewählt werden aus den Gattungen Eurypelma, Limulus, Astacus, Carcinus, Calliphora, Helix und Octopus.

3. Verfahren nach Anspruch 2, **dadurch gekennzeichnet,** daß ein Haemocyanin aus Eurypelma californicum (Vogelspinne) verwendet wird.

4. Verfahren nach Anspruch 2, **dadurch gekennzeichnet,** daß ein Haemocyanin aus Helix pomatia (Weinbergschnecke) verwendet wird.

5. Verfahren nach Anspruch 2, **dadurch gekennzeichnet,** daß ein Haemocyanin aus Octopus vulgaris (Krake) verwendet wird.

6. Verfahren nach Anspruch 2, **dadurch gekennzeichnet,** daß ein Haemocyanin aus Astacus fluviatilis (Flußkrebs) verwendet wird.

7. Verfahren nach Anspruch 2, **dadurch gekennzeichnet,** daß ein Haemocyanin aus Carcinus meanas (Strandkrabbe) verwendet wird.

8. Verfahren nach Anspruch 2, **dadurch gekennzeichnet,** daß ein Haemocyanin aus Limulus polyphemus (Pfeilschwanz) verwendet wird.

9. Verfahren nach Anspruch 2, **dadurch gekennzeichnet,** daß ein Arylphorin aus Calliphora erythrocephala verwendet wird.

10. Verfahren nach mindestens einem der vorhergehenden Ansprüche, **dadurch gekennzeichnet,** daß Untereinheiten eines Haemocyanins oder Arylphorins, vorzugsweise solche, die antigene Domänen enthalten, verwendet werden.

11. Verfahren nach Anspruch 10, **dadurch gekennzeichnet,** daß Untereinheiten eine der globulären Domänen der Haemocyanine der Mollusken mit einem Molekulargewicht von etwa 55.000 sind.

12. Verfahren nach Anspruch 10, **dadurch gekennzeichnet,** daß die Untereinheiten des Haemocyanins oder Arylphorins der Arthropoden Molekulargewichte von etwa 70.000 bis 80.000 aufweisen.

13. Verfahren nach Anspruch 12, **dadurch gekennzeichnet,** daß die Untereinheiten $\underline{a}$, $\underline{d}$ und/oder $\underline{e}$ des Haemocyanins aus Eurypelma californicum verwendet werden.

14. Verfahren nach Anspruch 13, **dadurch gekennzeichnet,** daß ein Peptidfragment, bestehend aus 23 Aminosäuren, verwendet wird, das durch chymotryptische Spaltung der Untereinheit $\underline{a}$ erhalten wird.

**15.** Verfahren nach Anspruch 14, **dadurch gekennzeichnet,** daß das Peptidfragment, bestehend aus 23 Aminosäuren, an Haemocyanin aus Carcinus oder Octopus oder an Arylphorin aus Calliphora gebunden ist.

**16.** Verfahren nach Anspruch 12, **dadurch gekennzeichnet,** daß die Domänen d und/oder a des βc-Haemocyanins aus Helix pomatia verwendet werden.

**17.** Verfahren nach mindestens einem der vorhergehenden Ansprüche, **dadurch gekennzeichnet,** daß das Haemocyanin und/oder Arylphorin mit Viren oder Virenbestandteilen, Bakterien oder Bakterienbestandteilen, Pilzen oder Pilzbestandteilen oder tierischen Parasiten oder deren Bestandteilen Strukturähnlichkeiten aufweisen oder mit diesen gemischt oder chemisch oder physikalisch gekoppelt werden.

**18.** Verfahren nach mindestens einem der vorhergehenden Ansprüche, **dadurch gekennzeichnet,** daß vor Applikation eine physikalische oder chemische Kopplung des Haemocyanins oder Arylphorins mit Antigenen zur Verstärkung der Antitumorwirkung erfolgt.

**19.** Verfahren nach Anspruch 18, **dadurch gekennzeichnet,** daß die Kopplung an Meth-A-Aszites-Fragmenten erfolgt.

**Claims**
**Claims for the following Contracting States : AT, BE, CH, DE, FR, GB, GR, IT, LI, LU, NL, SE**

**1.** Use of haemocyanin, with the exception of keyhole-limpet haemocyanin, and/or aryl phorin for the manufacture of a medicinal product for the treatment of tumours.

**2.** Use according to claim 1, **characterised in that** the haemocyanin or aryl phorin is isolated from tissues of molluscs or arthropods, the molluscs or arthropods being selected from the genera Eurypelma, Limulus, Astacus, Carcinus, Calliphora, Helix and Octopus.

**3.** Use according to claim 2, **characterised in that** a haemocyanin from Eurypelma californicum (bird spider) is used.

**4.** Use according to claim 2, **characterised in that** a haemocyanin from Helix pomatia (edible snail) is used.

**5.** Use according to claim 2, **characterised in that** a haemocyanin from Octopus vulgaris (common octopus) is used.

**6.** Use according to claim 2, **characterised in that** a haemocyanin from Astacus fluviatilis (crayfish) is used.

**7.** Use according to claim 2, **characterised in that** a haemocyanin from Carcinus meanas (shore crab) is used.

**8.** Use according to claim 2, **characterised in that** a haemocyanin from Limulus polyphemus (Atlantic horseshoe crab) is used.

**9.** Use according to claim 2, **characterised in that** an aryl phorin from Calliphora erythrocephala is used.

**10.** Use according to at least one of the preceding claims, **characterised in that** sub-units of a haemocyanin or aryl phorin, preferably those containing antigenic domains, are used.

**11.** Use according to claim 10, **characterised in that** sub-units are one of the globular domains of the haemocyanins of molluscs with a molecular weight of about 55 000.

**12.** Use according to claim 10, **characterised in that** the sub-units of the haemocyanin or aryl phorin of arthropods have molecular weights of about 70 000 to 80 000.

13. Use according to claim 12, **characterised in that** the sub-units a, d and/or e of the haemocyanin from Eurypelma californicum are used.

14. Use according to claim 13, **characterised in that** a peptide fragment consisting of 23 amino acids and obtained by the chymotryptic cleavage of sub-unit a is used.

15. Use according to claim 14, **characterised in that** the peptide fragment consisting of 23 amino acids is bound to haemocyanin from Carcinus or Octopus, or to aryl phorin from Calliphora.

16. Use according to claim 12, **characterised in that** domains d and/or a of the βc-haemocyanin from Helix pomatia are used.

17. Use according to at least one of the preceding claims, **characterised in that** the haemocyanin and/or aryl phorin show structural similarities to or are mixed with or chemically or physically bound to viruses or viral constituents, bacteria or bacterial constituents, fungi or fungal constituents, or animal parasites or their constituents.

18. Use according to at least one of the preceding claims, **characterised in that** prior to administration the haemocyanin or aryl phorin is physically or chemically bound to antigens to potentiate the anti-tumour effect.

19. Use according to claim 18, **characterised in that** the binding is to meth-A ascites fragments.

**Claims for the following Contracting State : ES**

1. Method for the manufacture of a medicinal product containing haemocyanin, with the exception of keyhole-limpet haemocyanin, and/or aryl phorin for the treatment of tumours, the method being performed as is in principle known.

2. Method according to claim 1, **characterised in that** the haemocyanin or aryl phorin is isolated from tissues of molluscs or arthropods, the molluscs or arthropods being selected from the genera Eurypelma, Limulus, Astacus, Carcinus, Calliphora, Helix and Octopus.

3. Method according to claim 2, **characterised in that** a haemocyanin from Eurypelma californicum (bird spider) is used.

4. Method according to claim 2, **characterised in that** a haemocyanin from Helix pomatia (edible snail) is used.

5. Method according to claim 2, **characterised in that** a haemocyanin from Octopus vulgaris (common octopus) is used.

6. Method according to claim 2, **characterised in that** a haemocyanin from Astacus fluviatilis (crayfish) is used.

7. Method according to claim 2, **characterised in that** a haemocyanin from Carcinus meanas (shore crab) is used.

8. Method according to claim 2, **characterised in that** a haemocyanin from Limulus polyphemus (Atlantic horseshoe crab) is used.

9. Method according to claim 2, **characterised in that** an aryl phorin from Calliphora erythrocephala is used.

10. Method according to at least one of the preceding claims, **characterised in that** sub-units of a haemocyanin or aryl phorin, preferably those containing antigenic domains, are used.

**11.** Method according to claim 10, **characterised in that** sub-units are one of the globular domains of the haemocyanins of molluscs with a molecular weight of about 55 000.

**12.** Method according to claim 10, **characterised in that** the sub-units of the haemocyanin or aryl phorin of arthropods have molecular weights of about 70 000 to 80 000.

**13.** Method according to claim 12, **characterised in that** the sub-units a, d and/or e of the haemocyanin from Eurypelma californicum are used.

**14.** Method according to claim 13, **characterised in that** a peptide fragment consisting of 23 amino acids and obtained by the chymotryptic cleavage of sub-unit a is used.

**15.** Method according to claim 14, **characterised in that** the peptide fragment consisting of 23 amino acids is bound to haemocyanin from Carcinus or Octopus, or to aryl phorin from Calliphora.

**16.** Method according to claim 12, **characterised in that** domains d and/or a of the $\beta$c-haemocyanin from Helix pomatia are used.

**17.** Method according to at least one of the preceding claims, **characterised in that** the haemocyanin and/or aryl phorin show structural similarities to or are mixed with or chemically or physically bound to viruses or viral constituents, bacteria or bacterial constituents, fungi or fungal constituents, or animal parasites or their constituents.

**18.** Method according to at least one of the preceding claims, **characterised in that** prior to administration the haemocyanin or aryl phorin is physically or chemically bound to antigens to potentiate the anti-tumour effect.

**19.** Method according to claim 18, **characterised in that** the binding is to meth-A ascites fragments.

**Revendications**
**Revendications pour les Etats contractants suivants : AT, BE, CH, DE, FR, GB, GR, IT, LI, LU, NL, SE**

**1.** Utilisation de l'hémocyanine, à l'exception de l'hémocyanine de gastéropode au sommet de coquille perforé, et/ou de l'arylphorine pour fabriquer un médicament pour le traitement des tumeurs.

**2.** Utilisation selon la revendication 1, caractérisée par le fait que l'on isole l'hémocyanine ou l'arylphorine des tissus de mollusques ou d'arthropodes, les mollusques ou les arthropodes étant choisis parmi les genres eurypelma, limulus, astacus, carcinus, calliphora, hélix et octopus.

**3.** Utilisation selon la revendication 2, caractérisée par le fait que l'on emploie une hémocyanine extraite du eurypelma californicum (araignée aviculariidae).

**4.** Utilisation selon la revendication 2, caractérisée par le fait que l'on utilise une hémocyanine extraite de hélix pomatia (escargot de Bourgogne).

**5.** Utilisation selon la revendication 2, caractérisée par le fait que l'on utilise une hémocyanine extraite de octopus vulgaris (poulpe).

**6.** Utilisation selon la revendication 2, caractérisée par le fait que l'on utilise une hémocyanine extraite de astacus fluviatilis (écrevisse).

**7.** Utilisation selon la revendication 2, caractérisée par le fait que l'on utilise une hémocyanine extraite de carcinus meanas (crabe de plage).

**8.** Utilisation selon la revendication 2, caractérisée par le fait que l'on utilise une hémocyanine extraite de limulus polyphemus (arthropode de la classe mérostomata, sous-classe xiphosura).

14

**9.** Utilisation selon la revendication 2 caractérisée par le fait que l'on utilise une arylphorine extraite de calliphora erythrocephala.

**10.** Utilisation selon au moins l'une des revendications précédentes caractérisée par le fait que l'on utilise des sous-divisions d'une hémocyanine ou d'une arylphorine, de préférence celles qui contiennent les régions antigènes.

**11.** Utilisation selon la revendication 10, caractérisée par le fait que les sous-divisions sont l'une des régions globulaires de l'hémocyanine des mollusques d'un poids moléculaire d'environ 55.000.

**12.** Utilisation selon la revendication 10, caractérisée par le fait que les sous-divisions de l'hémocyanine ou de l'arylphorine des arthropodes présentent des poids moléculaires d'environ 70.000 à 80.000.

**13.** Utilisation selon la revendication 12, caractérisée par le fait que l'on utilise les sous-divisions a, d et/ou e de l'hémocyanine extraite de Eurypelma californicum.

**14.** Utilisation selon la revendication 13, caractérisée par le fait que l'on utilise un fragment peptidique, constituée de 23 amino-acides, que l'on obtient par dissociation de la sous-division a par la chymotrypsine.

**15.** Utilisation selon la revendication 14, caractérisée par le fait que le fragment peptidique, constitué de 23 amino-acides est lié à une hémocyanine extraite de carcinus ou octopus ou à une arylphorine extraite de calliphora.

**16.** Utilisation selon la revendication 12, caractérisée par le fait que l'on utilise les régions d ou a de la βc-hémocyanine extraite de helix pomatia.

**17.** Utilisation selon au moins l'une des revendications précédentes, caractérisée par le fait que l'hémocyanine et/ou l'arylphorine présentent des similitudes de structure avec des virus ou des constituants de virus, des bactéries ou des constituants de bactéries, des champignons ou des constituants de champignons, ou des parasites animaux ou leurs constituants, ou bien sont mélangés avec ceux-ci ou bien leur sont couplés chimiquement ou physiquement.

**18.** Procédé selon au moins l'une des revendications précédentes, caractérisé par le fait qu'avant application, on procède à un couplage physique ou chimique de l'hémocyanine ou de l'arylphorine avec des antigènes pour renforcer l'action antitumorale.

**19.** Procédé selon la revendication 18, caractérisé par le fait que le couplage se fait sur des fragments de méth-A-ascites.

**Revendications pour l'Etat contractant suivant : ES**

**1.** Procédé de fabrication d'un médicament, contenant de l'hémocyanine, à l'exception de l'hémocyanine du gastéropode au sommet de coquille perforé, et/ou de l'arylphorine, pour le traitement des tumeurs, le procédé étant mis en oeuvre d'une façon connue en soi.

**2.** Procédé selon la revendication 1, caractérisé par le fait que l'on isole l'hémocyanine ou l'arylphorine des tissus de mollusques ou d'arthropodes, les mollusques ou les arthropodes étant choisis parmi les genres eurypelma, limulus, astacus, carcinus, calliphora, hélix et octopus.

**3.** Procédé selon la revendication 2, caractérisé par le fait que l'on emploie une hémocyanine extraite du eurypelma californicum (araignée aviculariidae).

**4.** Procédé selon la revendication 2, caractérisé par le fait que l'on utilise une hémocyanine extraite de hélix pomatia (escargot de Bourgogne).

**5.** Procédé selon la revendication 2, caractérisé par le fait que l'on utilise une hémocyanine extraite de octopus vulgaris (poulpe).

6. Procédé selon la revendication 2, caractérisé par le fait que l'on utilise une hémocyanine extraite de astacus fluviatilis (écrevisse).

7. Procédé selon la revendication 2, caractérisé par le fait que l'on utilise une hémocyanine extraite de carcinus meanas (crabe de plage).

8. Procédé selon la revendication 2, caractérisé par le fait que l'on utilise une hémocyanine extraite de limulus polyphemus (arthropode de la classe mérostomata, sous-classe xiphosura).

9. Procédé selon la revendication 2 caractérisé par le fait que l'on utilise une arylphorine extraite de calliphora erythrocephala.

10. Procédé selon au moins l'une des revendications précédentes caractérisé par le fait que l'on utilise des sous-divisions d'une hémocyanine ou d'une arylphorine, de préférence celles qui contiennent les régions antigènes.

11. Procédé selon la revendication 10, caractérisé par le fait que les sous-divisions sont l'une des régions globulaires de l'hémocyanine des mollusques d'un poids moléculaire d'environ 55.000.

12. Procédé selon la revendication 10, caractérisé par le fait que les sous-divisions de l'hémocyanine ou de l'arylphorine des arthropodes présentent des poids moléculaires d'environ 70.000 à 80.000.

13. Procédé selon la revendication 12, caractérisé par le fait que l'on utilise les sous-divisions $\underline{a}$, $\underline{d}$ et/ou $\underline{e}$ de l'hémocyanine extraite de Eurypelma californicum.

14. Procédé selon la revendication 13, caractérisé par le fait que l'on utilise un fragment peptidique, constituée de 23 amino-acides, que l'on obtient par dissociation de la sous-division $\underline{a}$ par la chymotrypsine.

15. Procédé selon la revendication 14, caractérisé par le fait que le fragment peptidique, constitué de 23 amino-acides est lié à une hémocyanine extraite de carcinus ou octopus ou à une arylphorine extraite de calliphora.

16. Procédé selon la revendication 12, caractérisé par le fait que l'on utilise les régions $\underline{d}$ ou $\underline{a}$ de la $\beta$c-hémocyanine extraite de helix pomatia.

17. Procédé selon au moins l'une des revendications précédentes, caractérisé par le fait que l'hémocyanine et/ou l'arylphorine présentent des similitudes de structure avec des virus ou des constituants de virus, des bactéries ou des constituants de bactéries, des champignons ou des constituants de champignons, ou des parasites animaux ou leurs constituants, ou bien sont mélangés avec ceux-ci ou bien leur sont couplés chimiquement ou physiquement.

18. Utilisation selon au moins l'une des revendications précédentes, caractérisée par le fait qu'avant application, on procède à un couplage physique ou chimique de l'hémocyanine ou de l'arylphorine avec des antigènes pour renforcer l'action antitumorale.

19. Utilisation selon la revendication 18, caractérisée par le fait que le couplage se fait sur des fragments de méth-A-ascites.